# EUROPEAN PATENT APPLICATION

(11) **EP 3 705 044 A1**
(43) Date of publication of application: **09.09.2020**
(21) Application number: 19161518.6
(22) Date of filing: 08.03.2019
(51) Int. Cl.: A61B 6/00, G01N 23/041

(54) **SYSTEM FOR X-RAY DARK FIELD; PHASE CONTRAST AND ATTENUATION TOMOSYNTHESIS IMAGE ACQUISITION**

(71) Applicant: Koninklijke Philips N.V., 5656 AG Eindhoven (NL)
(72) Inventor: KOEHLER, Thomas, 5656 AE Eindhoven (NL); YAROSHENKO, Andriy, 5656 AE Eindhoven (NL); BRENDEL, Bernhard Johannes, 5656 AE Eindhoven (NL)
(74) Representative: de Haan, Poul Erik

(57) **Abstract**

The present invention relates to a system (10) for X-ray dark field, phase contrast and attenuation tomosynthesis image acquisition. The system comprises an X-ray source (20), an interferometer arrangement (30), an X-ray detector (40), a control unit (50), and an output unit. A first axis is defined extending from a centre of the X-ray source to a centre of the X-ray detector. An examination region is located between the X-ray source and the X-ray. The first axis extends through the examination region, and the examination region is configured to enable location of an objection to be examined. The interferometer arrangement is located between the X-ray source and the X-ray detector. The interferometer arrangement comprises a first grating (32) and a second grating (34). A second axis is defined that is perpendicular to a plane that is defined with respect to a centre of the first grating and/or a centre of the second grating. The control unit is configured to control movement of the X-ray source and/or movement of the X-ray detector to provide a plurality of image acquisition states, wherein the X-ray source and X-ray detector are configured to operate to acquire image data. For each of the plurality of image acquisition states the first axis extends through the examination region at a different angle. The control unit is configured to control movement of the first grating or movement of the second grating in a lateral position direction perpendicular to the second axis. For each of the acquisition states the first grating or second grating is at a different lateral position of a plurality of lateral positions. The output unit is configured to output one or more of: dark field image data, phase contrast image data, and attenuation image data.

## Description

### FIELD OF THE INVENTION

The present invention relates to a system for X-ray dark field, phase contrast and attenuation tomosynthesis image acquisition, to a method for X-ray dark field, phase contrast and attenuation tomosynthesis image acquisition, as well as to a computer program element and a computer readable medium.

### BACKGROUND OF THE INVENTION

X-ray phase-contrast and dark-field imaging are two new imaging modalities that have shown the potential to significantly increase the diagnostic accuracy for soft-tissue imaging. One of the areas that has been identified to likely benefit most from these two new imaging modalities is chest radiography. It has been shown for example that X-ray dark-field information could significantly help diagnose such pulmonary disorders as COPD or fibrosis.

For the acquisition of these new imaging modalities a two or three-grating interferometer is introduced into the X-ray beam, normally termed G0, G1 and G2 gratings. The source grating G0, can be used to make radiation from the source more coherent but is not always necessary, and gratings G1 and G2 are normally termed phase and analyzer gratings. Subsequently, one of the two gratings G1 or G2 is moved perpendicular to the grating lamellae relative to the other gratings in a number of steps (so-called stepping), and if the source grating G0 is utilized it can be this grating that is stepped laterally. Thereby, for each new grating position an image is recorded. Comparison of the image sequence acquired with and without a sample in the beam, allows to calculate the three imaging signals: transmission or attenuation (conventional X-ray image), phase-contrast image, and dark-field image. At least three images in the sequence (stepping curve) are required in order to calculate the three imaging signals. However, in practice, significantly more images are recorded to allow for a stable signal extraction.

The acquisition of X-ray dark-field and phase-contrast information (referred to as DAX) thus relies on the acquisition of a series of images with different inter-grating positions. Due to the mechanical movement of the grating and the detector readout, this implies that the acquisition time is higher than for a conventional chest radiography. At the same time the resulting image is only a 2D radiography that suffers from often insufficient quantitative character. In particular, a short-coming of 2D DAX is that there is a sensitivity gradient along the optical axis of the system. Thus quantitative images are hard - if not impossible - to obtain.

There is a need to address these issues.

### SUMMARY OF THE INVENTION

It would be advantageous to have improved means of acquiring dark field, phase contrast, and attenuation X-ray image data.

The object of the present invention is solved with the subject matter of the independent claims, wherein further embodiments are incorporated in the dependent claims. It should be noted that the following described aspects and examples of the invention apply also to the system for X-ray dark field, phase contrast and attenuation tomosynthesis image acquisition, to the method for X-ray dark field, phase contrast and attenuation tomosynthesis image acquisition, as well as for the computer program element and computer readable medium.

In a first aspect, there is provided a system for X-ray dark field, phase contrast and attenuation tomosynthesis image acquisition. The system comprises:
- an X-ray source;
- an interferometer arrangement;
- an X-ray detector;
- a control unit; and
- an output unit.

A first axis is defined extending from a centre of the X-ray source to a centre of the X-ray detector. An examination region is located between the X-ray source and the X-ray. The first axis extends through the examination region, and the examination region is configured, or is of a size due to the relative locations of other parts of the system, to enable location of an object to be examined. The interferometer arrangement is located between the X-ray source and the X-ray detector. The interferometer arrangement comprises a first grating (32) and a second grating (34). A second axis is defined that is perpendicular to a plane that is defined with respect to a centre of the first grating and/or a centre of the second grating. The control unit is configured to control movement of the X-ray source and/or movement of the X-ray detector to provide a plurality of image acquisition states for which the X-ray source and X-ray detector are configured to operate to acquire image data. For each of the plurality of image acquisition states the first axis extends through the examination region at a different angle. The control unit is configured to control movement of the first grating or movement of the second grating in a lateral position direction perpendicular to the second axis. For each of the acquisition states the first grating or second grating is at a different lateral position of a plurality of lateral positions. The output unit is configured to output one or more of: dark field image data, phase contrast image data, and attenuation image data.

In other words, an X-ray imaging system is provided with an interferometric arrangement, where a first grating or second grating is stepped laterally in order to generate the required stepping curves from which dark field, phase contrast and attenuation image data can be reconstructed, and at the same time the X-ray source and detector are moved relative to each other in a tomosynthesis type movement. This lateral movement is perpendicular to an axis that extends perpendicularly to a tangent at the centre of the first grating or the second grating. Thus, the stepping curve data is acquired at different lines of site through an object, and reconstruction then provides for 2.5D or 3D dark field, phase contrast and attenuation image data rather than typical 2D data.

Also, in this manner the effects of parts of the object being either closer or further away from the second grating than other parts, that leads to higher or lower phase contrast or scattering signals in conventional dark field and phase contrast imaging, can be taken into account.

Based on this image acquisition a conventional radiography 2D image can also be synthesized that is more quantitative than conventional radiography, due to the fact that the distance dependency of the dark-field signal has been taken into account during image processing.

There is no significant increase in image acquisition time due to the required movement of the grating in the first place as compared to a "conventional" tomosynthesis acquisition. The patient is also not subjected to any additional radiation dose.

It is to be understood that the first grating can be flat and the second grating can be flat, and therefore the plane relating to the first grating is parallel to the plane of the flat grating, and the plane relating to the second grating is parallel to the plane of the flat grating. This, then defines the second axis that does not change its direction as one of the gratings is translated laterally. However, a grating can be curved, but the axis can similarly be defined and that does not change its direction as one of the gratings is translated laterally. For example, for a curved grating a plane can still be defined, with respect to the centre of the grating, where for example if that grating were to become gradually flatter it would become gradually more aligned with this plane.

In an example, the control unit is configured to implement an image processing algorithm to process the image data for the plurality of image acquisition states to generate the one or more: dark field image data, phase contrast image data, and attenuation image data.

In an example, the control unit is configured to implement the image processing algorithm to process the image data for the plurality of image acquisition states to generate one or more: tomosynthesis dark field image data, tomosynthesis phase contrast image data, and tomosynthesis attenuation image data.

In an example, the control unit is configured to control movement of the X-ray source and the X-ray detector to provide the plurality of image acquisition states, and the X-ray source is moved in an opposite direction to the X-ray detector to provide each of the plurality of image acquisition states.

In this manner, existing tomosynthesis systems that operate in this manner can be modified to include a DAX interferometric arrangement, where combined tomosynthesis-DAX image acquisitions can be used to provide 2.5D or 3D dark field, phase contrast and attenuation image data.

In an example, the control unit is configured to control movement of the interferometric arrangement, and for each of the acquisition states the interferometric arrangement is moved such that a relative orientation between the second axis and the first axis is maintained.

Thus, as the X-ray detector and X-ray source are moved such that the axis between them rotates or tilts, the interferometric arrangement is moved globally to maintain the alignment, in addition to one of the gratings of the interferometric being stepped at the same time as part of DAX image data acquisition.

In an example, the second axis is maintained oriented parallel to the first axis.

In an example, the image data comprises image data for the plurality of acquisition states when no object is present in the examination region and comprises image data for the plurality of acquisition states when an object is present in the examination region.

In an example, during acquisition of the image data for the plurality of acquisition states when the object is present, the system is configured not to move the object.

In an example, the control unit is configured to control movement of the second grating in a step-wise manner in the lateral position direction perpendicular to the second axis.

In an example, for each image data acquisition following the first image data acquisition the control unit is configured to step the second grating in the lateral position direction to a different lateral position.

In an example, the control unit is configured to control movement of the first grating in a step-wise manner in the lateral position direction perpendicular to the second axis.

In an example, for each image data acquisition following the first image data acquisition the control unit is configured to step the first grating in the lateral position direction to a different lateral position.

In an example, for two subsequent image acquisition states the first grating or second grating is at two different lateral positions.

Thus, one arrangement can be where for each angular line of sight through the examination region a grating is stepped once enabling for semi-continuous motion of all moveable components in line with one another. However, for each angular line of sight the grating can be stepped twice.

In a second aspect, there is provided a method for X-ray dark field, phase contrast and attenuation tomosynthesis image acquisition, the method comprising:
a) orienting an X-ray source relative to an X-ray detector to define a first axis extending from a centre of the X-ray source to a centre of the X-ray detector;
b) locating an examination region between the X-ray source and the X-ray, wherein the first axis extends through the examination region, and wherein the examination region is configured to enable location of an objection to be examined;
c) locating an interferometric arrangement between the X-ray source and the X-ray detector, wherein the interferometer arrangement comprises a first grating and a second grating, and wherein a second axis is defined that is perpendicular to a plane that is defined with respect to a centre of the first grating and/or the centre of the second grating;
d) controlling by a control unit movement of the X-ray source and/or movement of the X-ray detector and providing a plurality of image acquisition states for which the X-ray source and X-ray detector operate to acquire image data, and wherein for each of the plurality of image acquisition states the first axis extends through the examination region at a different angle;
e) controlling by the control unit movement of the first grating or movement of the second grating in a lateral position direction perpendicular to the second axis, and positioning for each of the acquisition states the first grating or second grating at a different lateral position of a plurality of lateral positions; and
h) outputting by an output unit one or more of: dark field image data, phase contrast image data, and attenuation image data.

According to another aspect, there is provided a computer program element controlling a system as previously described which, if the computer program element is executed by a processing unit, is adapted to perform the method steps as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a system for X-ray dark field, phase contrast and attenuation tomosynthesis image acquisition;
Fig. 2 shows a method for X-ray dark field, phase contrast and attenuation tomosynthesis image acquisition;
Fig. 3 shows a schematic set up of an example of a phase-contrast, dark-field and attenuation imaging system;
Fig. 4 shows stepping curve data obtained by the imaging system of Fig. 3; and
Fig. 5 shows Ground Truth (GT) data and reconstruction data for phase-contrast, dark field, and attenuation imaging utilising the described method.

### DETAILED DESCRIPTION OF EMBODIMENTS

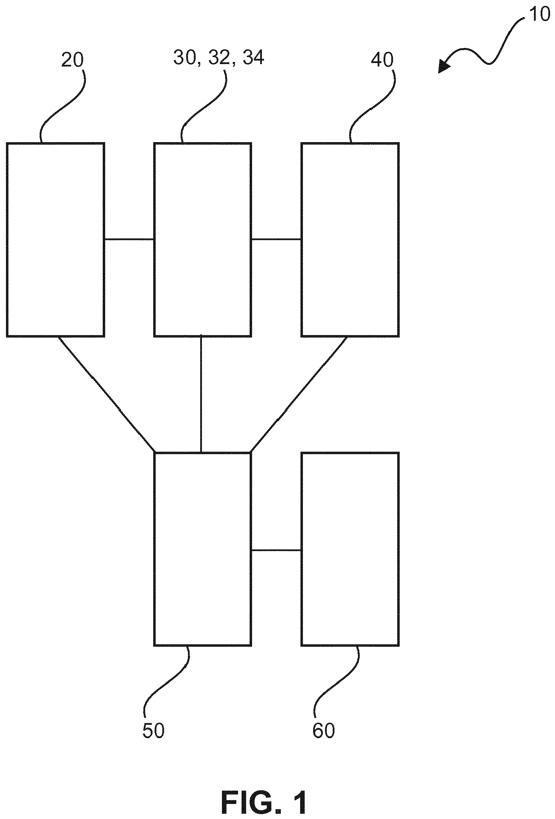

Fig. 1 shows an example of a system 10 for X-ray dark field, phase contrast and attenuation tomosynthesis image acquisition. Essential features are shown in solid lines and optional features are shown in hashed lines. The system comprises an X-ray source 20, an interferometer arrangement 30, an X-ray detector 40, a control unit 50, and an output unit 60. A first axis is defined extending from a centre of the X-ray source to a centre of the X-ray detector. An examination region is located between the X-ray source and the X-ray. The first axis extends through the examination region. Parts of the system are spaced from each other such that the examination region is configured, or sized, to enable location of an objection to be examined. The interferometer arrangement is located between the X-ray source and the X-ray detector. The interferometer arrangement comprises a first grating 32 and a second grating 34. A second axis is defined that is perpendicular to a plane that is defined with respect to a centre of the first grating and/or the centre of the second grating. The control unit is configured to control movement of the X-ray source and/or movement of the X-ray detector to provide a plurality of image acquisition states for which the X-ray source and X-ray detector are configured to operate to acquire image data. For each of the plurality of image acquisition states the first axis extends through the examination region at a different angle. The control unit is configured also to control movement of the first grating in a lateral position direction perpendicular to the second axis or control movement of the second grating in the lateral position direction perpendicular to the second axis. For each of the acquisition states the first grating or second grating is at a different lateral position of a plurality of lateral positions. The output unit is configured to output one or more of: dark field image data, phase contrast image data, and attenuation image data.

In an example, movement of the first grating or movement of the second grating in a lateral position direction perpendicular to the axis is also perpendicular to grating lines in the grating.

In an example, the first grating is located between the second grating and the X-ray source.

In an example, the examination region is located between the first grating and the X-ray source.

In an example, the interferometer arrangement comprises three gratings, where a source grating is located to interact with X-rays emitted from the source, and the source grating acts to increase the coherence of the X-ray that propagate through the interferometer arrangement. Thus, without the source grating there can be two gratings, where the first grating is closest to the source and is an absorption grating or a phase grating, and the second grating is closest to the detector and is an absorption grating. However, with three gratings the source grating closest to the source can be the first grating and either of the other two gratings can be the second grating, or the grating above that can be an absorption grating or phase grating can be the first grating etc.

In an example, the first grating is an absorption grating and the second grating is an absorption grating. In an example, the first grating is a phase grating and the second grating is an absorption grating.

According to an example, the control unit is configured to implement an image processing algorithm to process the image data for the plurality of image acquisition states to generate the one or more: dark field image data, phase contrast image data, and attenuation image data.

According to an example, the control unit is configured to implement the image processing algorithm to process the image data for the plurality of image acquisition states to generate one or more: tomosynthesis dark field image data, tomosynthesis phase contrast image data, and tomosynthesis attenuation image data.

According to an example, the control unit is configured to control movement of the X-ray source and the X-ray detector to provide the plurality of image acquisition states, wherein the X-ray source is moved in an opposite direction to the X-ray detector to provide each of the plurality of image acquisition states.

According to an example, the control unit is configured to control movement of the interferometric arrangement, wherein for each of the acquisition states the interferometric arrangement is moved such that a relative orientation between the second axis and the first axis is maintained.

Thus, as the X-ray detector and X-ray source are moved such that the axis between them rotates, the interferometric arrangement is moved globally to maintain the alignment, in addition to one of the gratings of the interferometric being stepped at the same time as part of DAX image data acquisition.

According to an example, the second axis is maintained oriented parallel to the first axis.

According to an example, the image data comprises image data for the plurality of acquisition states when no object is present in the examination region and comprises image data for the plurality of acquisition states when an object is present in the examination region.

According to an example, during acquisition of the image data for the plurality of acquisition states when the object is present, the system is configured not to move the object.

According to an example, the control unit is configured to control movement of the second grating in a step-wise manner in the lateral position direction perpendicular to the second axis.

According to an example, for each image data acquisition following the first image data acquisition the control unit is configured to step the second grating in the lateral position direction to a different lateral position.

According to an example, the control unit is configured to control movement of the first grating in a step-wise manner in the lateral position direction perpendicular to the second axis.

According to an example, for each image data acquisition following the first image data acquisition the control unit is configured to step the first grating in the lateral position direction to a different lateral position.

According to an example, for two subsequent image acquisition states the first grating or second grating is at two different lateral positions.

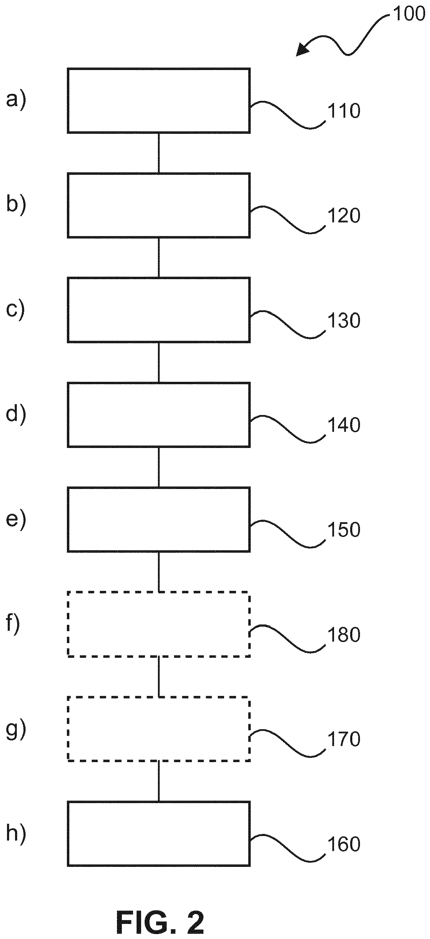

Fig. 2 shows a method 100 for X-ray dark field, phase contrast and attenuation tomosynthesis image acquisition in its basic steps where essential steps and optional steps are shown in hashed lines. The method comprises:
- in an orienting step 110, also referred to as step a), orienting an X-ray source 20 relative to an X-ray detector 40 to define a first axis extending from a centre of the X-ray source to a centre of the X-ray detector;
- in a locating step 120, also referred to as step b), locating an examination region between the X-ray source and the X-ray, wherein the first axis extends through the examination region, and wherein the examination region is configured to enable location of an objection to be examined;
- in a locating step 130, also referred to as step c), locating an interferometric arrangement 30 between the X-ray source and the X-ray detector, wherein the interferometer arrangement comprises a first grating 32 and a second grating 34, and wherein a second axis is defined that is perpendicular to a plane that is defined with respect to a centre of the first grating and/or the centre of the second grating;
- in a controlling step 140, also referred to as step d), controlling by a control unit 50 movement of the X-ray source and/or movement of the X-ray detector and providing a plurality of image acquisition states for which the X-ray source and X-ray detector operate to acquire image data, wherein for each of the plurality of image acquisition states the first axis extends through the examination region at a different angle;
- in a controlling step 150, also referred to as step e), controlling by the control unit movement of the first grating or movement of the second grating in a lateral position direction perpendicular to the second axis, and positioning for each of the acquisition states the first grating or second grating at a different lateral position of a plurality of lateral positions; and
- in an outputting step 160, also referred to as step h), outputting by an output unit 60 one or more of: dark field image data, phase contrast image data, and attenuation image data.

In an example, the method comprises step g) implementing 170 by the control unit an image processing algorithm to process the image data for the plurality of image acquisition states to generate the one or more: dark field image data, phase contrast image data, and attenuation image data.

In an example, step g) comprises implementing the image processing algorithm to process the image data for the plurality of image acquisition states to generate one or more: tomosynthesis dark field image data, tomosynthesis phase contrast image data, and tomosynthesis attenuation image data.

In an example, step d) comprises controlling movement of the X-ray source and the X-ray detector to provide the plurality of image acquisition states, wherein the X-ray source is moved in an opposite direction to the X-ray detector to provide each of the plurality of image acquisition states.

In an example, the method comprises step f) controlling 180 by the control unit movement of the interferometric arrangement, wherein for each of the acquisition states the interferometric arrangement is moved such that a relative orientation between the second axis and the first axis is maintained.

In an example, step f) comprises maintaining the second axis parallel to the first axis.

In an example, the method comprises acquiring image data for the plurality of acquisition states when no object is present in the examination region and comprises acquiring image data for the plurality of acquisition states when an object is present in the examination region.

In an example, the method comprises not moving the object during acquisition of the image data for the plurality of acquisition states when the object is present, the system is configured not to move the object.

In an example, step e) comprises controlling movement of the second grating in a step-wise manner in the lateral position direction perpendicular to the second axis. I n an example, step e) comprises, for each image data acquisition following the first image data acquisition, stepping the second grating in the lateral position direction to a different lateral position.

In an example, step e) comprises controlling movement of the first grating in a step-wise manner in the lateral position direction perpendicular to the second axis. In an example, step e) comprises for each image data acquisition following the first image data acquisition stepping the first grating in the lateral position direction to a different lateral position.

In an example, step e) comprises, for two subsequent of the image acquisition states, positioning the first grating or second grating at two different lateral positions.

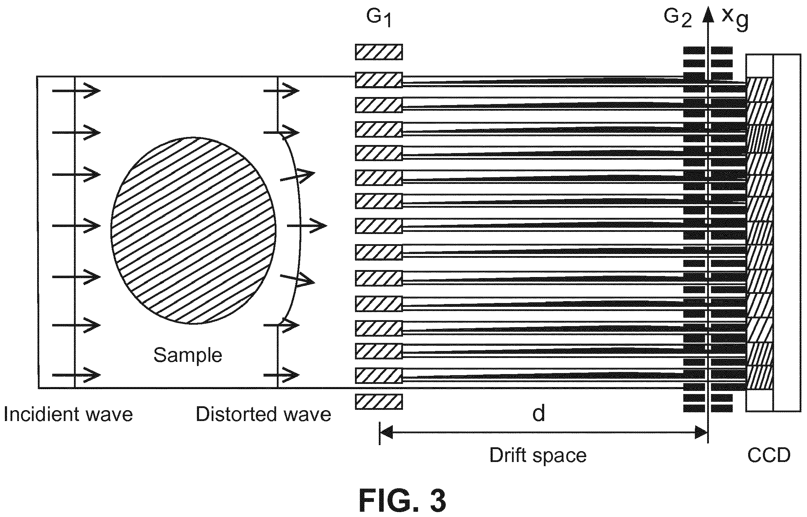

The system and method for X-ray dark field, phase contrast and attenuation tomosynthesis image acquisition are now described in more detail below for a specific example, and where reference is made to Figs. 3-5.

As discussed above, in the new imaging modality the inter-grating movement required for the stepping curve from which X-ray dark-field and phase-contrast information (along with normal attenuation information) can be determined is combined with a tomosynthesis-like movement of the X-ray source and detector around a patient (or just the X-ray source or detector can be moved). Thus, a new image of the sequence is acquired for a new relative position of the gratings and a new position of the source and detector with respect to the patient. This is then combined with an advanced image processing algorithm such as for example IBSIR, discussed below, to obtain a tomosynthesis image data for the dark field, phase contrast, and attenuation signals. This has important implications. The main advantages are summarized below:
- 2.5-dimensional information is generated, instead of 2D radiography imaging only;
- During image processing the variation of the dark-field (and phase-contrast) signal due to the depth (distance) dependency can be taken into account,
- Based on this image acquisition also a conventional radiography 2D image can be synthesized that is more quantitative than conventional radiography, due to the fact that the distance dependency of the dark-field signal has been taken into account during image processing;

- There is no significant increase in image acquisition time due to the required movement of the grating in the first place as compared to a "conventional" tomosynthesis acquisition;
- No additional X-ray dose is required.

The new imaging modality is explained with reference to Figs. 3-5.

Fig. 3 shows an example of the interferometer part of the system that can acquire X-ray phase contrast, dark field and attenuation image data. Reference to interferometer arrangement above, refers only to the gratings of the interferometer part of the system. The system is capable of imaging for the spatial distribution of attenuation of, or in, the sample and also at the same time capable of imaging for the spatial distribution of refraction (phase-contrast imaging) and also capable of imaging for the spatial distribution of small angle scattering (dark-field imaging). The system has a grating based interferometer. In this example, the interferometer comprises two grating structures G1 and G2, although in other examples a three grating interferometer (having gratings G0, G1 and G2) is used, where the source grating G0 near to the source is used to increase the coherence of radiation propagating through the sample and G1 and G2 gratings.

In Fig. 3, the source grating G0 is not shown and the discussion that follows considers a two grating structure G1 and G2, although all three of G0, G1 and G2 can be present, and where G0 can be the grating that is moved laterally. In Fig. 3 the grating G1 is a phase grating (but also can be an absorption grating) whereas G2 is an absorption gating. The system further comprises an X-ray source and the X-ray detector. The X-ray detector, here shown as a CCD detector, can be a 2D full view X-ray detector, which is either planar or curved. A plurality of detector pixels are arranged in rows and columns as an array to form a 2D X-ray radiation sensitive surface capable of registering X-ray radiation emitted by the X-ray source. The X-ray detector and the X-ray source are spaced apart to form an examination region. The examination region is suitably spaced to receive the sample to be imaged. The sample can be for example a patient's breast, or a patient's chest in order to examine the lung. Either of G1 or G2 can be curved or flat, but even if curved a plane can be defined that is parallel to the centre of the grating.

The sample then modulates attenuation, refraction, and small angle scattering information onto the radiation, which can then be extracted by operation of the grating tandem G1 and G2. The gratings G1, G2 induce an interference pattern, which can be detected at the X-ray detector as fringes of a Moire pattern. If there was no object in the examination region, there would still be an interference patter observable at the X-ray detector, called the reference pattern which is normally captured during a calibration procedure. This comes about by especially adjusting or "de-tuning" the mutual spatial relationship between the two gratings G1 and G2 by inducing a slight flexure for instance so that the two gratings are not perfectly parallel. Now, if the sample is positioned in the examination region and interacts with the radiation as mentioned, the Moire pattern, which is now more appropriately called the sample pattern, can be understood as a disturbed version of the reference pattern.

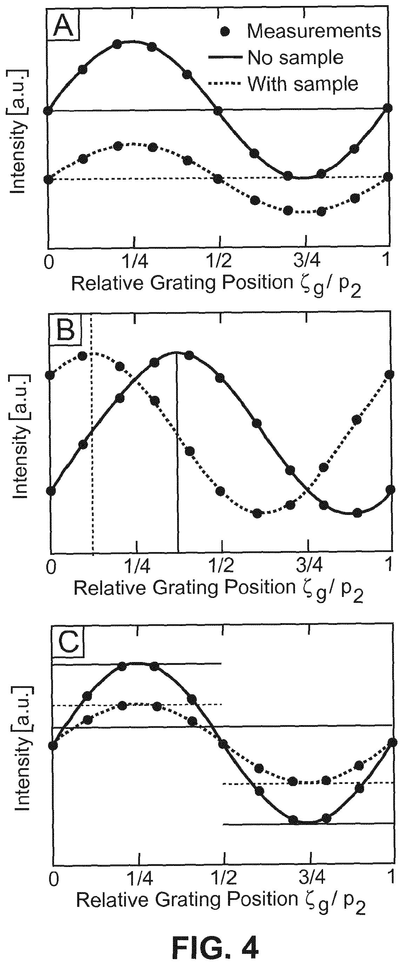

To separate this phase information from other contributions to the signal, such as attenuation by the sample, inhomogeneous illumination or imperfections of the gratings, the phase-stepping approach is utilized. One of the gratings (either G1 or G2 - or if G0 is present it could be stepped) is scanned along the transverse direction *x*_{g} (as shown in Fig. 3) over at least one period of the grating, and for every point of the scan an image is taken. If the source grating G0 is present, it can be this grating that is scanned along the transverse direction. The resultant phase contrast, dark-field, and attenuation data then oscillates sinusoidal, with and without the sample, as shown in Fig. 4 for phase-contrast (A), dark field (B), and attenuation (C). Further detail on the phase stepping approach can be found in the paper by Weitkamp et al, Optics Express, Vol. 13, No. 16, (2005) 6296-6304.

However, for the currently described system an additional movement step is carried out in a specific detailed example. This is that at each step of the grating G2 movement (as discussed above it could be G1 being stepped), the source and/or detector are moved in opposite directions with the interferometer grating arrangement as a whole moved to provide a new line of sight through the sample. The movement direction is transverse to an axis extending from the centre of the X-ray source to the centre of the detector, and as the detector and source move laterally, the longitudinal distance between them can remain the same. Then at the next step of grating G2, the source and detector are again moved in opposite directions, again with the interferometer grating arrangement as whole moved to provide another line of sight through the sample. In other words, for each step of a step approach for the determination of phase contrast, dark field, and attenuation image data, the source and detector (and indeed the whole interferometer arrangement) are rotated around the patient to provide a new line of sight through the patient in a tomosynthesis type protocol. However, the detector and source need not both move together. As discussed above the present image acquisition methodology can be carried out where the detector is stationary and the source moves to change a line of sight through the object, or the source is stationary and the detector is moved to change a line of sight through the object. However, in this specific example, both the detector and source can be moved together in opposite directions.

Further details on the tomosynthesis technique can be found in the paper by Dobbins and Godfrey: Phys. Med. Biol. Vol. 48 (2003) R65-R106.

Thus, in summary the new imaging acquisition proceeds as follows:
- A first image of the stepping curve is acquired with a certain inter-grating relative position and a certain relative position of the X-ray source, detector and the interferometer with respect to the patient;
- Subsequently a grating is moved relatively to the other grating or with respect to the other two gratings if there are three gratings. During this time the source, the interferometer, and the detector are moved relatively to the patient to a different position;
- Then a second image of the stepping curve is acquired;
- Subsequently the procedure is repeated until enough angular data for a tomosynthesis reconstruction is acquired.

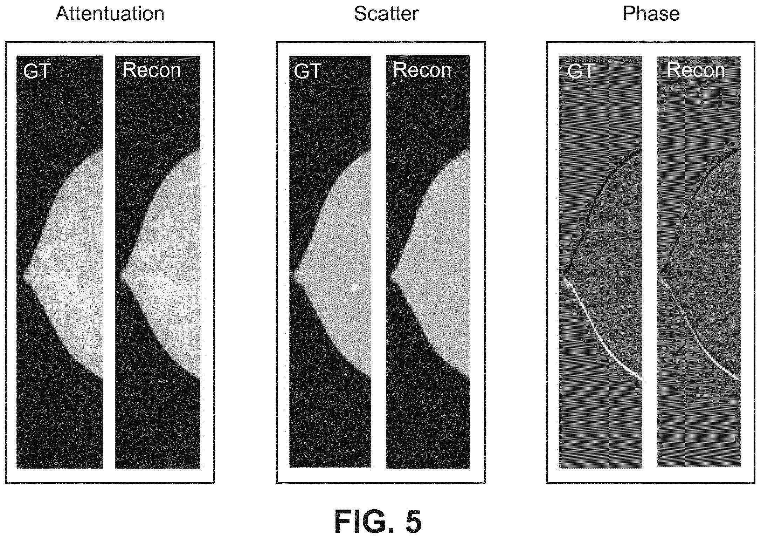

Subsequently an Intensity Based Statistical Iterative Reconstruction (IBSIR) technique is used to reconstruct the three imaging modalities. Further detail on IBSIR can be found in the paper by Brendel et al: Med. Phys. Vol. 43, (2016) 188-194. Other appropriate reconstruction algorithms could be used, for example utilizing sliding window phase retrieval as discussed in the paper by Zanette et al: PNAS, Vol. 109, No. 26, (2012) 10199-10204, if used as a tomographic acquisition. The IBSIR reconstruction results are shown in Fig. 5, which demonstrates the feasibility of performing combined phase stepping/tomosynthesis data acquisition as described above. In this case, a breast sample data was simulated and subsequently reconstructed. GT stands for a Ground Truth slice through the phantom, and Recon stands for the reconstructed tomosynthesis slice at the same position for attenuation, dark field and phase contrast images. This highlights the efficacy of the new imaging modality to provide tomosynthesis 2.5D or 3D data for dark field, phase contrast and attenuation X-ray image data.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A system (10) for X-ray dark field, phase contrast and attenuation tomosynthesis image acquisition, the system comprising:
- an X-ray source (20);
- an interferometer arrangement (30);
- an X-ray detector (40);
- a control unit (50); and
- an output unit (60);
wherein, a first axis is defined extending from a centre of the X-ray source to a centre of the X-ray detector;
wherein, an examination region is located between the X-ray source and the X-ray, wherein the first axis extends through the examination region, and wherein the examination region is configured to enable location of an objection to be examined;
wherein, the interferometer arrangement is located between the X-ray source and the X-ray detector, wherein the interferometer arrangement comprises a first grating (32) and a second grating (34), and wherein a second axis is defined that is perpendicular to a plane that is defined with respect to a centre of the first grating and/or a centre of the second grating;
wherein, the control unit is configured to control movement of the X-ray source and/or movement of the X-ray detector to provide a plurality of image acquisition states for which the X-ray source and X-ray detector are configured to operate to acquire image data, and wherein for each of the plurality of image acquisition states the first axis extends through the examination region at a different angle;
wherein, the control unit is configured to control movement of the first grating or movement of the second grating in a lateral position direction perpendicular to the second axis, and wherein for each of the acquisition states the first grating or second grating is at a different lateral position of a plurality of lateral positions; and
wherein, the output unit is configured to output one or more of: dark field image data, phase contrast image data, and attenuation image data.

2. System according to claim 1, wherein the control unit is configured to implement an image processing algorithm to process the image data for the plurality of image acquisition states to generate the one or more: dark field image data, phase contrast image data, and attenuation image data.

3. System according to claim 2, wherein the control unit is configured to implement the image processing algorithm to process the image data for the plurality of image acquisition states to generate one or more: tomosynthesis dark field image data, tomosynthesis phase contrast image data, and tomosynthesis attenuation image data.

4. System according to any of claims 1-3, wherein the control unit is configured to control movement of the X-ray source and the X-ray detector to provide the plurality of image acquisition states, wherein the X-ray source is moved in an opposite direction to the X-ray detector to provide each of the plurality of image acquisition states.

5. System according to any of claims 1-4, wherein the control unit is configured to control movement of the interferometric arrangement, wherein for each of the acquisition states the interferometric arrangement is moved such that a relative orientation between the second axis and the first axis is maintained.

6. System according to claim 5, wherein the second axis is maintained oriented parallel to the first axis.

7. System according to any of claims 1-5, wherein the image data comprises image data for the plurality of acquisition states when no object is present in the examination region and comprises image data for the plurality of acquisition states when an object is present in the examination region.

8. System according to claim 7, wherein during acquisition of the image data for the plurality of acquisition states when the object is present, the system is configured not to move the object.

9. System according to any of claims 1-8, wherein the control unit is configured to control movement of the second grating in a step-wise manner in the lateral position direction perpendicular to the second axis.

10. System according to claim 9, wherein for each image data acquisition following the first image data acquisition the control unit is configured to step the second grating in the lateral position direction to a different lateral position.

11. System according to any of claims 1-8, wherein the control unit is configured to control movement of the first grating in a step-wise manner in the lateral position direction perpendicular to the second axis.

12. System according to claim 11, wherein for each image data acquisition following the first image data acquisition the control unit is configured to step the first grating in the lateral position direction to a different lateral position.

13. System according to any of claims 1-12, wherein for two subsequent image acquisition states the first grating or second grating is at two different lateral positions.

14. A method (100) for X-ray dark field, phase contrast and attenuation tomosynthesis image acquisition, the method comprising:
a) orienting (110) an X-ray source (20) relative to an X-ray detector (40) to define a first axis extending from a centre of the X-ray source to a centre of the X-ray detector;
b) locating (120) an examination region between the X-ray source and the X-ray, wherein the first axis extends through the examination region, and wherein the examination region is configured to enable location of an objection to be examined;
c) locating (130) an interferometric arrangement (30) between the X-ray source and the X-ray detector, wherein the interferometer arrangement comprises a first grating (32) and a second grating (34), and wherein a second axis is defined that is perpendicular to a plane that is defined with respect to a centre of the first grating and/or a centre of the second grating;
d) controlling (140) by a control unit (50) movement of the X-ray source and/or movement of the X-ray detector and providing a plurality of image acquisition states for which the X-ray source and X-ray detector operate to acquire image data, and wherein for each of the plurality of image acquisition states the first axis extends through the examination region at a different angle;
e) controlling (150) by the control unit movement of the first grating or movement of the second grating in a lateral position direction perpendicular to the second axis, and positioning for each of the acquisition states the first grating or second grating at a different lateral position of a plurality of lateral positions; and
h) outputting (160) by an output unit (60) one or more of: dark field image data, phase contrast image data, and attenuation image data.

15. A computer program element for controlling a system according to any of claims 1-13, which when executed by a processor is configured to carry out the method of claim 14.
